Europäisches Patentamt

European Patent Office

Office européen des brevets

(19)

(11) Veröffentlichungsnummer: **0 049 385**
**B1**

## EUROPÄISCHE PATENTSCHRIFT

(12)

(45) Veröffentlichungstag der Patentschrift:
08.05.85

(21) Anmeldenummer: 81107282.6

(22) Anmeldetag: 16.09.81

(51) Int. Cl.⁴: **C 08 J 9/36,** C 08 J 3/12,
C 12 N 11/04

(54) Perlpolymerisat und dessen Verwendung zur Immobilisierung von Enzymen.

(30) Priorität: 02.10.80 DE 3037198

(43) Veröffentlichungstag der Anmeldung:
14.04.82 Patentblatt 82/15

(45) Bekanntmachung des Hinweises auf die Patenterteilung:
08.05.85 Patentblatt 85/19

(84) Benannte Vertragsstaaten:
AT BE CH DE FR GB IT LI LU NL SE

(56) Entgegenhaltungen:
DE - B - 1 274 128
FR - A - 2 369 290

DIE ANGEWANDTE MAKROMOLEKULARE CHEMIE,
Band 97, 24. Juni 1981, Hüthig & Wepf Verlag, Seiten
179-187, Heidelberg, DE, P. SCHMIDT et al.: "Zum
Einfluss von Ultraschall auf die Oberflächenporosität
von Polystyrol-Enzym-Trägern"

Die Akte enthält technische Angaben, die nach dem
Eingang der Anmeldung eingereicht wurden und die
nicht in dieser Patentschrift enthalten sind.

(73) Patentinhaber: BASF Aktiengesellschaft,
Carl-Bosch-Strasse 38, D-6700 Ludwigshafen (DE)

(72) Erfinder: Tschang, Chung-Ji, Dr.,
Bürgermeister-Kropp-Strasse 7, D-6702 Bad Dürkheim
(DE)
Erfinder: Sanner, Axel, Dr., Lorscher Ring 2c,
D-6710 Frankenthal (DE)
Erfinder: Marcinowski, Stefan, Dr.,
Kopernikusstrasse 49, D-6700 Ludwigshafen (DE)

**0 049 385**

## Beschreibung

Die vorliegende Erfindung betrifft ein makroporöses, vernetztes Perlpolymerisat und dessen Verwendung zur Immobilisierung von Enzymen.

Immobilisierte, d. h. trägergebundene Enzyme, finden u. a. Anwendung in der medizinischen Analytik, bei der Herstellung pharmazeutischer Produkte, der Herstellung optisch aktiver Substanzen sowie bei der Herstellung von Nahrungsmitteln, wie beispielsweise die Isoglucose. Die Vorteile der Verwendung von immobilisierten Enzymen ergeben sich aus ihrer Wiederverwendbarkeit, ihrer einfachen Abtrennung vom Substrat bzw. dessen Lösung, ihrer oftmals erhöhten Stabilität im Vergleich zur löslichen Form, aus der Vermeidung einer Verunreinigung der Reaktionsprodukte sowie aus der Möglichkeit, kontinuierliche Reaktionen in Säulen oder ähnlichen Reaktoren durchführen zu können.

Es sind eine Reihe von Möglichkeiten der Immobilisierung von Enzymen bekannt. Die bekannten Verfahren weisen jedoch mehr oder weniger erhebliche Mängel auf. So erhält man bei der Einbettung von Enzymen durch Polymerisation in der Regel gelartige Präparate mit mangelhaften mechanischen Eigenschaften. Außerdem besteht bei diesem Verfahren die Gefahr einer Schädigung der Enzyme durch Monomere oder, wenn auch in geringerem Ausmaß, durch Präpolymere. Schließlich gelingt die Einbettung der Enzyme oft nicht vollständig, so daß ein Teil derselben aus den Präparaten bei Gebrauch ausblutet.

Bei den gebräuchlichsten Verfahren zur Immobilisierung von Enzymen erfolgt die Herstellung einer kovalenten Bindung an einen Träger. Dabei kann als Trägermaterial sowohl anorganisches als auch organisches polymeres Material nativen oder synthetischen Ursprungs verwendet werden. Die Bindung des Enzyms erfolgt in der Regel mit Hilfe von reaktiven Gruppen, die unter milden Bedingungen mit Hydroxy- und/oder Aminogruppen der Enzyme zu reagieren vermögen. Eine ausführliche Übersicht befindet sich in Methods in Enzymology, Vol. XLIV: Immobilized Enzymes, Academic Press, 1976. Trotz der Vielzahl der bekannten derartigen Verfahren erhält man in jedem Falle ein Präparat, welches einen oder mehrere der folgenden Nachteile aufweist: mangelhafte Aktivität, geringe mechanische Stabilität, unbefriedigende hydrodynamische Eigenschaften, Empfindlichkeit gegen mikrobiellen Abbau, komplizierte und teure Herstellung des Trägers, geringe Lagerstabilität des unbeladenen Trägers durch empfindliche reaktive Gruppen sowie das Vorhandensein von toxischen Substanzen auf dem Träger, die zur kovalenten Bindung der Enzyme an den Träger benötigt werden. Darüber hinaus sind kovalent bindende Träger nicht oder nur mit unverhältnismäßig großem Aufwand regenerierbar.

Als weiteres Immobilisierungsverfahren wird die Adsorption von Enzymen an anorganische oder organische Träger vorgeschlagen, wobei als Ausgangsstoffe oft Materialien nativen Ursprungs verwendet werden. Beispiele für derartige Träger sind poröse Oxide von Magnesium und Aluminium und DEAE-Cellulose (DEAE = Diethylaminoethyl). Die Adsorption und Desorption von Enzymen an sogenannte Adsorbentien, d. h. makroporöse Harze, zum Zwecke der Reinigung wird in der DE-PS 1 274 128 vorgeschlagen. Im allgemeinen gilt die adsorptive Bindung von Enzymen als wenig dauerhaft, so daß mit dem Ausbluten der Enzyme zu rechnen ist (J. Chibata: Immobilized Enzymes, Kodansha Ltd./John Wiley & Sons, 1978).

Der Erfindung liegt die Aufgabe zugrunde, poröse organische polymere Träger zu schaffen, die die obengenannten Nachteile vermeiden und Enzyme unter weitgehender Erhaltung ihrer Aktivität zu binden vermögen, so daß die entstehenden enzymatisch aktiven Präparate unter den üblichen Bedingungen der Anwendung stabil sind. Die erfindungsgemäßen Präparate weisen als besondere Vorzüge hohe Aktivität, gute hydrodynamische Eigenschaften und mechanische Stabilität auf und sind einfach herzustellen.

Gegenstand der Erfindung ist ein makroporöses, vernetztes Perpolymerisat, welches aus 10 bis 100% Divinylbenzol und/oder einem drei oder mehr Vinylgruppen enthaltenden Benzol sowie 0 bis 90% Styrol, das ein- oder mehrfach $C_{1-4}$-alkyl-substituiert sein kann, und/oder Methylmethacrylat hergestellt und das dadurch gekennzeichnet ist, daß die Oberflächen der Perlen einer gesonderten, einen Abrieb erzeugenden Behandlung unterzogen worden sind.

Es ist möglich, weitere mit Styrol und Divinylbenzol copolymerisierbare Monomere in einer Menge von bis zu 30% bezogen auf die Gesamtmenge an Monomeren, einzupolymerisieren. Solche Monomere sind z. B. Alkylester der Methacrylsäure, Alkylester der Acrylsäure, sowie Acryl- und Methacrylsäure selbst. Allerdings nimmt mit zunehmender Kettenlänge der Monomeren die Brauchbarkeit ab. Auch der Ersatz des Divinylbenzols durch mehrfunktionelle Ester, wie Hexandioldimethacrylat oder Trimethylolpropantriacrylat, führt zu keinen brauchbaren Polymerisaten. Die Brauchbarkeit eines Monomeren läßt sich am besten durch einen Vorversuch ermitteln. Es ist auch zu berücksichtigen, daß wasserlösliche Substanzen — wie die Acryl- und Methacrylsäure — bei der Suspensionspolymerisation nicht vollständig in das Polymerisat eingebaut werden. Diese müssen deshalb in überschüssiger Menge in die Polymerisationsreaktion eingesetzt werden.

Die Polymerisation des Monomerengemischs wird in Form einer Suspensionspolymerisation mit Wasser als äußerer Phase durchgeführt, wobei eine Teilchengröße von 0,05 bis 3 mm, bevorzugt 0,2 bis 0,8 mm, angestrebt wird. Zur Erzeugung der Makroporosität des Polymerisats wird ein Porenbildner verwendet. Als Porenbildner eignen sich beispielsweise Alkane mit 7 bis 12 C-Atomen, bevorzugt n-Octan oder Benzine mit einem Siedepunkt $\geqq 100°$C. Die Menge an Porenbildner beträgt 50 bis

2

0 049 385

400 Gew.-%, bezogen auf die Monomeren. Die obere Grenze der Porenbildner-Menge ergibt sich aus den mechanischen Eigenschaften des Polymerisats und ist vom jeweiligen Porenbildner abhängig. Wesentlich ist die Erzeugung eines Porenvolumens von 0,8 bis 4 cm$^3$/g, bevorzugt 1,5 bis 3,5 cm$^3$/g, wobei die Durchmesser der Poren 2,5 · 10$^{-5}$ bis 2 · 10$^{-3}$ mm, bevorzugt 4 · 10$^{-5}$ bis 5 · 10$^{-4}$ mm betragen sollen. Die Herstellung von makroporösen Perlpolymerisaten als solche ist bekannt und kann z. B. der US-Patentschrift 3 322 695 entnommen werden.

Zur Erzielung einer hohen immobilisierten Enzymaktivität wird der Träger einer gesonderten, einen Abrieb erzeugenden Behandlung unterzogen, wodurch vermutlich die Durchlässigkeit der Oberfläche des Trägers erhöht wird. Geeignete Maßnahmen sind das Rollen des Polymerisats, gegebenenfalls in Gegenwart von harten, inerten Körpern, wie z. B. Glaskugeln, oder heftiges Schütteln in einer Maschine. Dabei kann das Polymerisat in trockener oder feuchter Form vorliegen. Die Dauer der Behandlung richtet sich nach ihrer Intensität. Eine weitere Methode zur Erhöhung der Aktivität ist das schnelle Rühren des Polymerisats in Wasser oder einer anderen Flüssigkeit, wobei eine Apparatur mit rauher Oberfläche verwendet wird und/oder ein hartes Inertmaterial, wie z. B. Sand, zugesetzt wird. Auch die Verwendung von Aeroklassierern, vorzugsweise Schwingsiebsichtern, stellt eine geeignete Maßnahme dar. Nach der Abrieb erzeugenden Behandlung ist es vorteilhaft, den Träger sorgfältig zu waschen. Die bei der Herstellung des Polymeren und dessen normalen Handhabung auftretenden Oberflächenbeanspruchungen reichen zur Erzielung des erfindungsgemäßen Effekts keineswegs aus.

Sofern der Träger nicht bereits in Wasser vorliegt, muß er vor der Beladung mit einem Enzym (oder mehreren Enzymen) aufgrund seines hydrophoben Charakters mit Wasser benetzt bzw. benetzbar gemacht werden. Hierzu wird er z. B. in ein organisches, mit Wasser mischbares Lösungsmittel, wie z. B. Methanol, Ethanol, Aceton, Dimethylformamid oder Tetrahydrofuran, gegeben. Aus diesem wird er dann direkt oder nach Überführung in Wasser oder eine wäßrige Pufferlösung in die wäßrige Lösung des zu immobilisierenden Enzyms gegeben.

Die Beladung des Trägers geschieht in einer wäßrigen Enzymlösung, die gegebenenfalls einen Puffer enthalten kann. Die Konzentration des Enzyms in der Lösung beträgt 1 bis 100 mg/ml, die Beladungsdauer 1 bis 100 Stunden, vorzugsweise 5 bis 70 Stunden. Die Temperatur der Enzymlösung liegt — je nach Enzym — bei 0 bis 90°C. Nach der Beladung wird der Träger von überschüssigem Enzym durch Waschen befreit und als heterogener Katalysator mit enzymatischer Aktivität entweder im diskontinuierlichen Betrieb oder im kontinuierlichen, z. B. in einer Säule, eingesetzt.

Zur Behandlung auf dem Träger geeignete Enzyme sind vorzugsweise Invertase, Glucoseisomerase, Amyloglucosidase, $\alpha$- und $\beta$-Amylase, Aminosäureacylase, Penicillinacylase und Hydantoinase. Ferner sind geeignet: Oxidoreduktasen — wie Alkoholdehydrogenase, Lactatdehydrogenase, Aminosäureoxidase, Peroxidase, Katalase, Glucoseoxidase, Alkoholoxidase, Succinatdehydrogenase, Glutamatdehydrogenase, Uricase, Phenoloxidase, Catecholoxidase, Monoaminooxidase, Lipoxygenase, Luciferase, Nitratreduktase, Nitritreduktase, Chlorperoxidase, Acetaldehyddehydrogenase, Aldehydoxigenase, Diaphorase, Cholesterinoxidase, Glutarthioreduktase, Hydroxysteroiddehydrogenase, Xanthinoxidase, Dopaminhydroxylase, Cytochromoxidase, Monoaminooxidase, Diacetylreduktase, Superoxiddismutase, Limonatdehydrogenase — ; Transferasen — wie Polynucleotidphosphorylase, Dextransucrase, Phosphorylase, Carbamatkinase, Aminotransferase, Transaldolase, Methyltransferase, Pyruvatkinase, Carbamoyltransferase, Phosphofructokinase, Dextransynthetase — ; Hydrolasen — wie Lipase, Esterase, Lactase, Lysozym, Cellulase, Urease, Trypsin, Chymotrypsin, Glutaminase, Asparaginase, Papain, Ficin, Pepsin, Leucinaminopeptidase, Carboxypeptidase A + B, Naringinase, Bromelain, Subtilisin, Phospholipase, Isoamylase, Cephalosporinamidase, Adenosindeaminase, Penicillinase, Maltase, Dextranase, Desoxyribonuclease, Sulfatase, Pullulanase, Phosphatase, $\alpha$-Galactosidase, Dextranase, $\beta$-Glucanase — ; Lyasen — wie Tryptophanase, Tyrosindecarboxylase, Oxinitrilase, Phenylalanindecarboxylase, Phenylalaninammoniumlyase, Aminosäuredecarboxylase, Pyruvatdecarboxylase, Fumarase, Enolase, Aspartase, Aminolaevulindehydratase, Carboanhydratase — ; Isomerasen — wie Aminosäureracemase, Triosephosphatisomerase — ; Ligasen — wie Glutathionsynthetase — .

Zur Regenerierung kann der Träger mit Hilfe der wäßrigen Lösung eines oberflächenaktiven Mittels vom Enzym befreit werden. Danach wird durch Behandlung mit einem organischen Lösungsmittel, beispielsweise Methanol, das oberflächenaktive Mittel entfernt. Der Träger wird dann — wie oben beschrieben — erneut mit Enzym beladen und zum Einsatz gebracht.

Beispiel 1

A. Herstellung des Perlpolymerisats

Unter Verwendung von a) 50 g Styrol, b) 25 g Ethylvinylbenzol, c) 25 g Divinylbenzol, d) 100 g n-Octan und e) 500 ml Wasser sowie 1 g Lauroylperoxid als Radikalbildner und 1 g Polyvinylpyrrolidon als Suspensionshilfsmittel wird in einem Kolben mit Rückflußkühler und Rührer unter Einleiten von Stickstoff ein Perlpolymerisat hergestellt, wobei 4 Stunden auf 70°C und dann 4 Stunden auf 95°C erhitzt wird. Das Polymerisat wird nacheinander in Aceton, Wasser und Methanol gewaschen und danach bei 70°C im Vakuum getrocknet und auf eine Teilchengröße von 3,1 · 10$^{-3}$ bis 8 · 10$^{-3}$ cm

3

gesiebt, Ausbeute 83 g. Das Produkt besitzt folgende Porendaten, die quecksilberporosimetrisch ermittelt wurden: Porenvolumen: 1,96 cm³/g, Porendurchmesser: 4,6 · 10⁻⁶ bis 3 · 10⁻⁵ cm.

B. 20 g des gemäß A erhaltenen Polymerisats werden mit 4 g Glasperlen (Durchmesser 3 mm) 24 Stunden in einer 250 ml Glasflasche auf einem Rollstuhl gerollt. Nach Entfernen der Glasperlen wird das Polymerisat nacheinander jeweils 1 Stunde bei Raumtemperatur in Aceton, Wasser und Methanol gerührt und bei 70° C Vakuum getrocknet.

## C. Beladung des Polymerisats mit Enzym

0,5 g Polymerisat wird zweimal 1 Stunde in 50 ml 0,05 M Natriumacetatpuffer (pH 5,3) geschüttelt Anschließend wird es in 20 ml einer Lösung von 100 mg Invertase (150 Units/mg) in dem genannten Puffer gegeben und 64 Stunden bei 4° C geschüttelt. Danach wird das Polymerisat bei Raumtemperatur zweimal 5 Minuten und einmal 1 Stunde in je 50 ml 0,0025 M Natriumacetatpuffer (pH = 5,3) gewaschen.

Zur Ermittlung der Enzymaktivität (»immobilisierten Aktivität«) wird das Polymerisat in 50 ml einer Lösung von 17,5 g Sucrose in 0,001 M Natriumacetatpuffer (pH = 5,3) geschüttelt. Die Hydrolyse der Sucrose wird polarimetrisch ermittelt.

Die Beladung des gemäß A hergestellten Polymerisats führt zu einer immobilisierten Aktivität von 9,0 g Sucrose pro g Polymerisat und pro Stunde. Das gemäß B behandelte Polymerisat zeigt hingegen eine Aktivität von 32,2 g Sucrose pro g und pro Stunde.

## Beispiel 2

20 g gemäß Beispiel 1A hergestelltes Polymerisat werden in einer 250 ml Glasflasche 4 Stunden oder in einem 250 ml Kunststoffgefäß in Gegenwart von 6 g Stahlkugeln (4,5 mm Durchmesser) 0,5 Stunden geschüttelt. Anschließend werden die Produkte analog Beispiel 1B gewaschen und getrocknet und analog Beispiel 1C beladen. Die erhaltenen Produkte besitzen eine immobilisierte Aktivität von 30,5 g bzw. 32,7 g Sucrose pro g und pro Stunde.

## Beispiel 3

Das Beispiel 1 wird wiederholt, jedoch wird in A anstelle von n-Octan Benzin (Kp 155—185° C) eingesetzt. Das Polymere wird analog Beispiel 2 ohne Prallkörper behandelt. Die Beladung und Prüfung des Polymeren erfolgt analog Beispiel 1C. Die immobilisierte Aktivität beträgt 28,9 g Sucrose pro g und pro Stunde.

## Beispiel 4

Das Beispiel 1 wird wiederholt, jedoch wird in A anstelle von Styrol 75 g Methymethacrylat und anstelle von Ethylvinylbenzol und Divinylbenzol 25 g technisches Divinylbenzol verwendet. Die Beladung und Prüfung des Polymeren erfolgt analog Beispiel 1C. Die immobilisierte Aktivität beträgt 36,4 g Sucrose pro g und pro Stunde.

## Beispiel 5

Das Beispiel 1 wird wiederholt, jedoch werden als Monomere für die Polymerisation 100 g technisches Divinylbenzol verwendet. Der Träger enthält also etwa 50% Divinylbenzol und etwa 50% Ethylvinylbenzol. Das Trägermaterial wird auf eine Teilchengröße von 5 · 10⁻³ bis 6,3 · 10⁻³ cm gesiebt und 3 Stunden analog Beispiel 2 ohne Prallkörper behandelt. Die Beladung und Prüfung des Polymeren erfolgt analog Beispiel 1C. Die immobilisierte Aktivität beträgt 35,9 g Sucrose pro g und pro Stunde.

## Beispiel 6

Es wird ein Träger entsprechend Beispiel 1 hergestellt, wobei jedoch 20 g Glasperlen verwendet werden und sich die Roll-Behandlung über 96 Stunden erstreckt. 1 g dieses Trägers wird mit Invertase beladen (vgl. Beispiel 1C) und in eine Säule mit einem Durchmesser von 1,6 cm gefüllt, wobei sich eine Füllhöhe von ca. 2 cm ergibt. Die Säule wird auf 30° C thermostatisiert. Durch die Säule wird mit einer Fließgeschwindigkeit von 40 ml/Stunde eine Lösung von 400 g Sucrose/l in 0,001 M Natriumacetatpuffer (pH 5,3) gepumpt. Die Hydrolyse der Sucrose wird polarimetrisch verfolgt. Nach 1 Tag beträgt der Hydrolysegrad der Sucrose nach Passieren der Säule 89%, nach 20 Tagen 66%.

## Beispiel 7

0,2 g Träger — wie in Beispiel 6 verwendet — werden 64 Stunden bei 4°C in 20 ml Lösung von 100 mg ᴁ-Amylase (130 Units/mg) in 0,005 M Natriumacetatpuffer vom pH 6,0 geschüttelt. Dann wird der Träger jeweils bei Raumtemperatur zweimal 5 Minuten und zweimal 1 Stunde in je 50 ml 0,05 M Natriumacetatpuffer von pH 6,0 gewaschen. Zur Ermittlung der immobilisierten enzymatischen Aktivität wird der Träger bei 30°C 30 Minuten in 50 ml Lösung von 5 g Stärke nach Zulkowsky (Merck) in 0,016 M Natriumacetatpuffer (pH 6,0) geschüttelt. Die entstehenden Oligosaccharide werden mit 3,5-Dinitrosalicylsäure nach P. Berenfeld (Methods in Enzymology, Vol. I, 149, Academic Press, 1955), bestimmt. Aktivität des Trägers: 7,5 g hydrolysierte Stärke pro g und pro Stunde.

## Beispiel 8

0,2 g Träger — wie in Beispiel 6 verwendet — werden 64 Stunden bei 4°C in 20 ml Lösung von 100 mg ᴁ-Amylase (28 Units/mg) in 0,05 M Natriumacetatpuffer (pH 4,8) geschüttelt. Dann wird der Träger jeweils bei Raumtemperatur zweimal 5 Minuten und zweimal 1 Stunde in je 50 ml 0,05 M Natriumacetatpuffer von pH 4,8 gewaschen. Zur Ermittlung der immobilisierten enzymatischen Aktivität wird der Träger bei 30°C 30 Minuten in 50 ml Lösung von 5 g Stärke nach Zulkowsky (Merck) in 0,016 M Natriumacetatpuffer (pH 4,8) geschüttelt. Die gebildete Maltose wird mit 3,5-Dinitrosalicylsäure nach P. Berenfeld (Methods in Enzymology, Vol. 5, 149; Academic Press, 1955) bestimmt. Aktivität des Trägers: 3,3 g gebildete Maltrose pro g und pro Stunde.

## Beispiel 9

Analog Beispiel 8 wird Amyloglucosidase an den Träger gebunden. Aktivität des Trägers: 2,3 g gebildete Glucose pro g und pro Stunde.

## Beispiel 10

0,1 g Träger (vgl. Beispiel 1) werden 70 Stunden bei 4°C mit 10 ml Lösung von 50 mg Aminoacylase (25 Units/mg) in 0,05 M Phosphatpuffer (pH 7,6) geschüttelt. Dann wird der Träger jeweils bei Raumtemperatur zweimal 5 Minuten und einmal 1 Stunde in je 50 ml 0,05 M Phosphatpuffer von pH 7,6 gewaschen. Zur Ermittlung der immobilisierten enzymatischen Aktivität wird der Träger bei 30°C in 50 ml Lösung von 2,5 g N-Acetyl-D,L-methionin in 0,05 M Phosphatpuffer (pH 7,6) geschüttelt. Die Lösung ist außerdem $5 \cdot 10^{-4}$ M an Kobaltchlorid. Das gebildete L-Methionin wird photometrisch mit Ninhydrin bestimmt. Aktivität des Trägers: 1,7 g Methionin pro g und pro Stunde.

**Patentansprüche**

1. Makroporöses, vernetztes Perlpolymerisat, welches aus

10 — 100% Divinylbenzol und/oder einem mehr als zwei Vinylgruppen enthaltendem Benzol und
0 — 90% Styrol, das ein- oder mehrfach $C_{1-4}$-alkyl-substituiert sein kann und/oder Methylmethacrylat

hergestellt ist, dadurch gekennzeichnet, daß die Oberflächen der Perlen einer gesonderten, einen Abrieb erzeugenden Behandlung unterzogen worden sind.
2. Polymerisat gemäß Anspruch 1, dadurch gekennzeichnet, daß die Behandlung der Oberfläche durch Rollen des Polymerisats oder durch Schütteln erfolgt ist.
3. Polymerisat gemäß Anspruch 1, dadurch gekennzeichnet, daß die Behandlung der Oberfläche durch Rühren des Polymerisats in Gegenwart einer Flüssigkeit in einer Apparatur mit einer rauhen Wandung und/oder in Gegenwart eines harten inerten Materials erfolgt ist.
4. Polymerisat gemäß Anspruch 1, dadurch gekennzeichnet, daß die Behandlung der Oberfläche in einem Aeroklassierer erfolgt ist.
5. Verwendung der Polymerisate gemäß Anspruch 1—4 zur Immobilisierung von Enzymen.

## Claims

1. A mocroporous, crosslinked bead polymer which has been prepared from

10 to 100% of divinylbenzene and/or a benzene containing mor than two vinyl groups, and from
0 to 90% of styrene, which can be mono- or poly-C$_{1-4}$-alkyl-substituted and/or methyl methacrylate,

wherein the beads have been subjected to an abrasion-causing surface treatment.

2. A polymer as claimed in claim 1, wherein the surface treatment is carried out by tumbling the polymer, or by shaking.

3. A polymer as claimed in claim 1, wherein the surface treatment is carried out by stirring the polymer in the presence of a liquid in an apparatus having a rough wall and/or in the presence of a hard, inert material.

4. A polymer as claimed in claim 1, wherein the surface treatment is carried out in an air classifier.

5. The use of a polymer as claimed in claims 1 to 4 for immobilizing enzymes.

## Revendications

1. Polymérisat en suspension, réticulé, macroporeux, qui est préparé à partir de

10 — 100% de divinylbenzène et/ou un benzène contenant plus de deux groupes vinyle et
0 — 90% de styrène, qui peut être substitué une ou plusieurs fois par alkyle en C$_{1-4}$, et/ou du méthacrylate de méthyle

caractérisé par le fait que les surfaces des perles ont été soumises à un traitement spécial produisant une abrasion.

2. Polymérisat selon la revendication 1, caractérisé par le fait que le traitement des surfaces résulte de l'action de rouleaux sur le polymérisat ou de secousses.

3. Polymérisat selon la revendication 1, caractérisé par le fait que le traitement des surfaces résulte de l'agitation du polymérisat en présence d'un liquide, dans un appareil muni d'une paroi rugueuse et/ou en présence d'un matériau inerte, dur.

4. Polymérisat selon la revendication 1, caractérisé par le fait que le traitement des surfaces s'opère dans un aéro-séparateur.

5. Utilisation du polymérisat selon la revendication 1—4 pour l'immobilisation d'enzimes.